# EUROPEAN PATENT SPECIFICATION

(11) **EP 1 453 743 B1**
(45) Date of publication and mention of the grant of the patent: **23.07.2008**
(21) Application number: 02777539.4
(22) Date of filing: 15.11.2002
(51) Int. Cl.: B65D 81/26

(54) **PHARMACEUTICAL PRODUCT AND METHOD WITH AN ADSORBENT**
PHARMAZEUTISCHES PRODUKT UND VERFAHREN MIT ADSORBER
PRODUIT ET METHOD PHARMACEUTIQUES UTILISANT UN ADSORBANT

(30) Priority: 17.11.2001 GB 0127612; 12.12.2001 US 340705 P
(43) Date of publication of application: 08.09.2004
(73) Proprietor: Aventis Pharma Limited, West Malling, Kent ME19 4AH (GB)
(72) Inventor: BARKER, Frank c/o Aventis Pharma Limited, West Malling Kent ME19 4AH (GB); BASSO, Nils c/o Aventis Pharma Deutschland GmbH, 65926 Frankfurt am Main (DE)
(74) Representative: Jones, Stephen Anthony
(86) International application number: PCT/GB2002/005147
(87) International publication number: WO 2003/043905

(56) References cited:
- US-A- 5 300 138
- US-A- 5 657 748
- US-A- 5 911 937
- US-A- 6 103 141
- US-B1- 6 315 112
- US-B1- 6 316 521

## Description

### Field of the Invention

This invention relates to a method and a package for packaging medical devices comprising a medicament. More particularly, it relates to a package and packaging method that utilizes an adsorbent material, such as a molecular sieve, that adsorbs or absorbs a gaseous substance that gradually accumulates in the inner local environment of an impermeable package, so as to prevent formation of adducts due to chemical reactions between the medicament in the medical device and the trace gaseous substance.

### Background of the Invention

Medical devices usually need to be packed in substantially impermeable packages to prevent atmospheric moisture ingress. The use of such impermeable packages may cause accumulation of certain trace substances within the sealed local environment to a level sufficient for them to interact with the medicament contained in the medical device. Such interaction, for example, may result in an adduct between the medicament and the trace substance. For instance, a dry powder inhaler generally includes a number of plastic components molded from an acetal homopolymer, and the plastic components may contain trace formaldeyde formed as a breakdown product during the molding of acetal resins. It is believed that the trace formaldehye released from the plastic components is capable of forming an adduct with various medicaments when packaged within a substantially impermeable container.

Therefore, there is a need in the art for an improvement in substantially impermeable medical device packages for preventing trace substances from interacting with the medicament in the medical device.

### Summary of the Invention

A primary object of the present invention is to provide a new package for medical device comprising a medicament in which formation of adducts, such as medicament-polymer adducts, will be reduced or eliminated.

This and other objects of the present invention are attained by providing a package comprising (i) an outer substantially impermeable package; (ii) a medical device comprising a medicament that has a tendency to form adducts in the medicament; and (iii) an adsorbent material, preferably a molecular sieve. Both the medical device comprising a medicament and the adsorbent material are sealed within the package.

It is believed that the mechanism by which the adsorbent material prevents adduct formation is by entrapping residual gaseous substances released by various components of the medical device comprising a medicament so that those substances will not accumulate within the package to a significant level and interact with the medicament contained in the medical device to form the adducts. However, this explanation of the mechanism is not a limitation on the present invention and an adsorbent material may achieve its effect on adduct formation through other known or unknown mechanisms.

The various features of novelty which characterize the invention are pointed out with particularity in the claims annexed to and forming a part of this disclosure. For a better understanding of the invention, its operating advantages, and specific objects attained by its use, reference should be made to the drawings and the following description in which there are illustrated and described preferred embodiments of the invention.

### Brief Description of the Drawings

Figure 1 is a graph summarizing a study that shows that the molecular sieve is an effective adsorbent against formation of the medicament-polymer adduct Compound A in triamcinolone acetonide/lactose blends.
Figure 2 is a view, partially cut-away, of a typical dry-power inhaler package according to the present invention.
Figure 3 depicts two of a number of possible locations for the absorbent in a dry-power inhaler. For example, they could possibly be molded as part of one of the plastic components, or could be provided in a container that is fixed to the inhaler, eg by mechanical means, or by welding, or by use of an adhesive.

### Detailed Description of the Preferred Embodiments

It is appreciated that certain features of the invention, which are, for clarity, described in the context of separate embodiments, may also be provided in combination in a single embodiment, provided that said embodiment lies within the scope of the appended claims. Also, various features of the invention which are, for brevity, described in the context of a single embodiment, may also be provided separately or in any suitable subcombination, as long as said subcombination remains within the scope of the claims.

### The Problem of Adduct Formation

During a feasibility study of a dry powder inhaler (DPI) containing triamcinolone acetonide (TAA), an increasing level of the impurity from the synthesis, delta 14-TAA, was observed. This was first seen at the 6 week check point in samples stored at 40 °C/75%RH and was observed to the greatest extent in the 100 µg/actuation DPI with a level of delta 14-TAA (0.48 % w/w) which failed the specification limit of 0.40 % w/w. After obtaining this out-of-specification result, a series of investigation were performed. Using an HPLC method, it is discovered that a new peak was present that eluted just before delta 14-TAA. It was further determined that in the original stability study, this new peak co-eluted with delta 14-TAA and thus gave rise to the out-of-specification reading of delta 14-TAA. When using the HPLC method, the delta 14-TAA level remained constant, as expected for an impurity from the synthesis. The new peak, determined using liquid chromatography/mass spectrometry (LC/MS), was found to be corresponding to a mass of TAA plus 30, and had the same retention time as the product of the reaction between TAA and formaldehyde. Thus, it is believed that the new peak was the adduct between TAA and formaldehyde, which was identified as the C11 hydroxymethyl derivative of TAA and was assigned Compound A with the following structure:

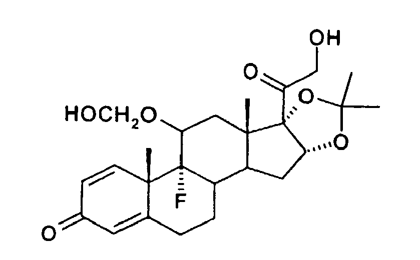

Compound A belongs to the glucocorticoid class of molecules, in which the class are known to possess anti-inflammatory activities and are commonly utilized for the treatment of numerous inflammatory diseases, for example asthma. The effects of compounds in treating asthma can be examined by any one of the procedures known in the art, for example those disclosed in I.L. Bernstein et al, Chest 81, 20 (1982); K. Florey, Anal. Profiles Drug Subs. 1, 397-421 (1972); and D. H. Seih, ibid. 11, 615-649 (1982)

The term "medical device" as used herein is intended to encompass any device that is capable of containing a medicament, wherein the device has a component that gradually releases a gaseous substance that may interact with the medicament to form an adduct. The device may be a substantially impermeable package so that any gaseous substance released from a component of the device may accumulate in the package and may react with the medicament to form an adduct. Also, the device may be adequately sealed such that the gaseous substance released from a component of the devise may accumulate in the device itself and may react with the medicament to form an adduct. Therefore, the invention is not limited to any specific type of medical devices or any specific medicaments they contain, as long as there is a potential to form one or more adducts due to the accumulation of one or more residual gaseous substances during storage in an impermeable package. Examples of such devices include, medicament loaded syringes, inhalation devices containing medicaments, for example, dry-powdered inhalers.

The term "medicament" as used herein is intended to encompass any medicament capable of being stored in a device and has a tendency to form one or more adducts during storage by reacting with a gaseous substance that is gradually released from a component of the device. A medicament "has a tendency to form one or more adducts" means that the medicament will form one or more adducts if no measure, such as inclusion of an adsorbent material within the package, is taken to prevent the adduct formation.

For example, the medicament can be any material that has a pharmaceutical effect as applied, including, but not limited to, antibiotics, antimicrobials, antiseptics, bacteriocins, bacteriostats, disinfectants, steroids, anesthetics, antifingal agents, anti-inflammatory agents, antibacterial agents, antiviral agents, antitumor agents, and tissue growth promoting substances. In one embodiment of the invention, the medicaments may be selected from, for example, analgesics, e.g. codeine, dihydromorphine, ergotamine, fentanyl or morphine, anginal preparations, e.g. diltiazem; antiallergics, e.g. cromoglycate, ketotifen or nedocromil; antiinfectives e.g. cephalosporins, penicillins, streptomycin, sulphonamides, tetracyclines pentamidine, and Neuraminidase Inhibitors, such as zanamivir (Relenza^{®}) available from GlaxoSmithkline; and Ribavirin (Virazole^{®}) manufactured by ICN Pharmaceuticals, Inc.; antihistamines, e.g. mnethapyfilene; antitussives, e.g. noscapine; beta-adrenergics that include bronchodilators such as salbutamol, salmeterol, ephedrine, adrenaline, fenoterol, forinoterol, isoprenaline, phenylephrine, phenylpropanolamine, reproterol, rimiterol, terbutaline, isoetharine, tulobuterol, orciprenaline, or (-)4-amino-3,5-dichloro-.alpha.-[[[6-[2-(2-pyridinyl)ethoxy]hexyl]-amino]m ethyl]benzenemethanol, epinephrine (Primatene), formoterol (Foradil), isoproterenol (Isuprel), isoetharine (Bronkosol), metaproterenol (Alupent, Metaprel), albuterol (Proventil, Ventolin), terbutaline (Bricanyl, Brethine), bitolterol (Tornalate), pirbuterol (Maxair), salmeterol (Serevent), salmeterol + fluticasone combination (Advair Diskus), and albuterol + atrovent combination (Combivent); sodium channel blockers such as amiloride, anticholinergics e.g. ipratropium, atropine or oxftropium; hormones, e.g. cortisone, hydrocordisone or prednisolone; and therapeutic proteins and peptides, e.g. insulin or glucagon; anti-inflammatory medicaments used in connection with the treatment of respiratory diseases include steroids such as NASACORT AQ^{®} (triamcinolone acetonide), AZMACORT AQ^{®} (triamcinolone acetonide) flunisolide, fluticasone, budesonide, triamcinolone acetonide, beclomethasone (Vanceril, Beclovent), budesonide (Pulmicort) dexamethasone, flunisolide (Aerobid), fluticasone (Flovent), salmeterol + fluticasone combination (Advair Diskus), and triamcinolone (Azmacort), and Mediator-release inhibitors such as Intal® (cromolyn sodium), and nedocromil sodium (Tilade); leukotrine (LT) inhibitors, vasoactive intestinal peptide (VIP), tachykinin antagonists, bradykinin antagonists, endothelin antagonists, heparin furosemide, anti-adhesion molecules, cytokine modulators, biologically active endonucleases, recombinant human (rh) DNase compounds, alpha-antitrypsin and disodium cromoglycate (DSCG); and lung surfactants such as lipid-containing compositions as described in TONGE et. Al, WO 99/09955; Pulmonary surfactants as decribed in Devendra et. Al, Respir Res 2002, 3:19; Infasurf^{®}available from ONY; Curosurf^{®}available from Dey Laboratories; Exosurf^{®} by Glaxo Wellcome; Survanta available from Abbot; Surfaxin^{®} lung surfactant available from Discovery Laboratories.

The term "component" is meant to encompass a component of a medical devise that undesirably releases a gaseous substance. In particular, a component comprising a polyacetal material (polyoxymethylene). Polyoxymethylene (polyacetal plastics- Trade Name: *Delrin (DuPont), Ultraform (the Ultraform Co.), and Hostaform (Ticona))* are a group of plastics produced by polymerizing formaldehyde. Polyoxymethylene is used in toiletry and cosmetic articles as well as medical devices such as inhalers, and syringes. A number of DPI device components are manufactured from polyacetal plastic that is known to contain residual formaldehyde formed during the molding process. Polyacetal is readily available from a number of commercial sources, for example Sigma-Aldrich, Milwaukee, WI 53201.

The term "package" as used herein is meant to encompass a container that is substantially impermeable to moisture and to a gaseous substance released from a component of the device. For example, the package may be made of metal, glass, or plastic, and is selected from the group consisting of bottles, bags, drum boxes, and irregularly shaped containers.

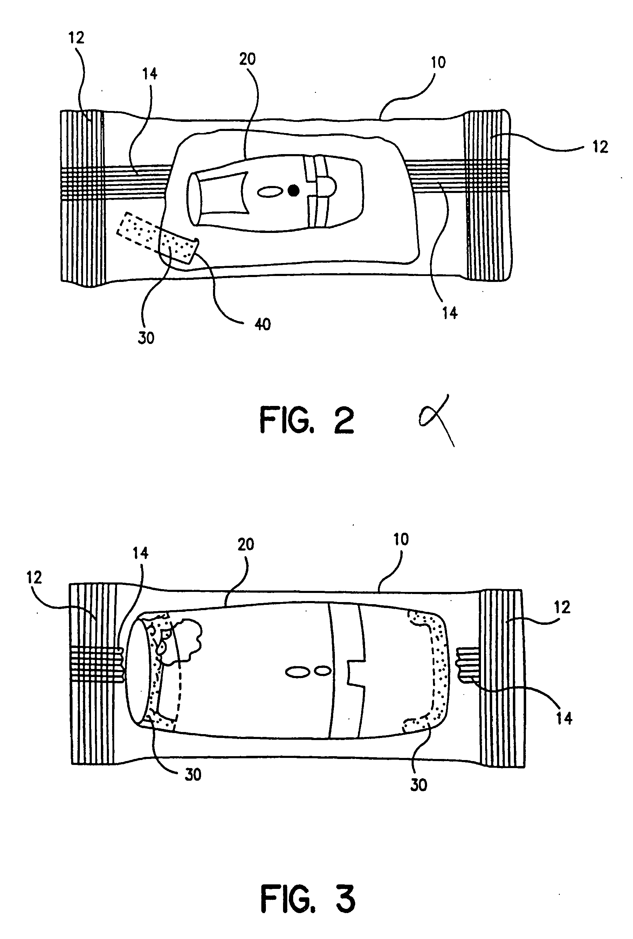

In one embodiment, the package is a conventional flexible package and its manufacturing is well within the knowledge of the people skilled in the art. In general, the flexible package is constructed from flat reels of laminate which are folded or otherwise formed according to the packaging equipment technology into a package by means of sealing and cutting. For example, as shown in figure 2, the package has a substantially impermeable flexible package 10, in which a dry powder inhaler 20 and a molecular sieve 30 enclosed in a porous sachet 40 are sealed. In this embodiment the package is constructed from a flat reel of flexible material which is curled around into a long tube and a seal 14 is formed by heating (welding) the edges of the tube together. The cross seals 12 are formed by a straight heater bar which clamps the laminate tube before and after the package contents (i.e., the inhaler and the adsorbent sachet). It also cuts the continuous tube into individual packs. As a result, there is a long continuous seal 14 down the middle of the pack and the cross seals 12 at both ends. Also, in figure 3, the package has a substantially impermeable flexible package 10, in which a dry powder inhaler 20 and adsorbent 30 are situated. The adsorbent 30 can be molded as part of one of the plastic components, or could be provided in a container that is fixed to the inhaler. In this embodiment the package is constructed from a flat reel of flexible material which is curled around into a long tube and a seal 14 is formed by heating (welding) the edges of the tube together. The cross seals 12 are formed by a straight heater bar that clamps the laminate tube before and after the package contents. It also cuts the continuous tube into individual packs. As a result, there is a long continuous seal 14 down the middle of the pack and the cross seals 12 at both ends.

Other package types may include more or less seals according to the desired shape of the container, which may be flat seals or crimped, and may include gussets. The seals may be formed by heating (welding) or by the use of pressure sensitive materials. In a further embodiment the flexible laminates may be formed using heat, pressure and/or vacuum into blisters or pockets to contain the product and which are then sealed by heating.

Although a flexible package is preferred, other types of enclosures or containers may be suitable, whether flexible or inflexible, provided that the enclosure chosen is substantially impermeable to moisture ingress. In general, when the package or enclosure is impermeable, or substantially impermeable, to moisture, it is also impermeable, or substantially impermeable, to the gaseous substance that has potential to interact with the medicament in the medical device.

A preferred flexible material for making the package is a laminate, although other materials may also be satisfactorily employed. The main limitation is that the package material must be substantially impermeable to atmosphere moisture.

The laminate used in making packages generally consists of several layers of materials either co-extruded or bonded together to form an apparently single film of "laminate". As an example, a suitable laminate may have three layers adhesively laminated to each other: an inner layer, a barrier layer and an outer layer. For example, Pharmaflex Ltd., part of Alcan inc. (Cramlington, Northumberland, England) supplies a laminate film having three layers: 12 micron polyester / 9 micron aluminum foil / 50 micron polyethylene (product catalog LMP-F BRI/72/H1).

The inner layer forms the inside of the package (in contact with the medical device) and is normally a thermoplastic layer and heat-sealable. A common material for the inner layer is polyethylene, but other polyolefinic or cyclo-olefinic materials may also be used. In addition, specialist materials such as ionomers are also frequently used for making the inner layer, for example, the ionomer under the tradename Surlyn.

The barrier layer is situated between the inner and outer layers and provides impermeability to the pack. Aluminum foil is commonly used for the barrier layer, although any other metals capable of being rolled into thin sheets can also be satisfactorily used. A typical thickness for the aluminum foil layer is about 8 or 9 microns. Alternatively, the barrier layer may be metalised films, made up of tin, iron, zinc, magnesium or other metals coated by vacuum deposition or sputtering onto a polymeric sheet.

The outer layer normally provides support, impact resistance, protection for the barrier layer and general robustness to the pack. A commonly used material for the outer layer is polyester, although other material, such as paper, may also be used. Most flexible laminate materials for packaging are commercially available. For example, Pharmaflex Ltd., part of Alcan inc. (Cramlington, Northumberland, England) supplies a laminate film having three layers: 12 micron polyester / 9 micron aluminum foil / 50 micron polyethylene (product catalog LMP-F BRI/72/H1).

The term "substantially impermeable to the gaseous substance" as used herein, means that the level of the gaseous substance in the enclosed volume of the package or enclosure will elevate if no measure, such as inclusion of an adsorbent material within the package or enclosure, is taken to reduce it. Or in other words, the egress rate of the gaseous substance allowed by the package or enclosure is lower than the rate by which it is released into the enclosed volume of the package or enclosure by the medical device components.

The present invention is intended to encompass the free acids, free bases, salts, amines and various hydrate forms including semi-hydrate forms of such medicaments and is particularly directed towards pharmaceutically acceptable formulations of such medicaments which are formulated in combination with pharmaceutically acceptable excipient materials generally known to those skilled in the art, preferably without other additives such as preservatives.

The medicament may be in the form of a solid, such as a powder or a solid film, or in the form of a liquid, such as a watery, viscous, or paste-like material. The medicament may also be compounded with a variety of additives, such as surfactants or emulsifiers, and vehicles.

Preferred medicament formulations do not include additional components such as preservatives which have a significant effect on the overall formulation. Thus preferred formulations consist essentially of pharmaceutically active medicament and a pharmaceutically acceptable carrier (e.g., water and/or ethanol). However, if a medicament is liquid without an excipient the formulation may consist essentially of the medicament that has a sufficiently low viscosity that it can be aerosolized using a dispenser of the present invention.

A preferred medicament formulation consists essentially of a medicament, or a physiologically acceptable salt or solvate thereof, optionally in combination with one or more other pharmacologically active agents.

Optionally, the formulations according to the invention may further comprise one or more cosolvent. A polar cosolvent such as C₂₋₆ aliphatic alcohols and polyols, e.g., glycerol, ethanol, isopropanol and propylene glycol, preferably ethanol, may be included in the medicament formulation in the desired amount, either as the only excipient or in addition to other excipients, such as surfactants. Suitably, the medicament formulation may contain 0.01 to 5% w/w based on the propellant of a polar cosolvent, e.g., ethanol, preferably 0.1 to 5% w/w, e.g., about 0.1 to 1% w/w.

Optionally, the formulations according to the invention may further comprise one or more surfactants. The surfactants must be physiologically acceptable upon administration by inhalation. Within this category are included surfactants such as oleic acid, sorbitan trioleate, sorbitan mono-oleate, sorbitan monolaurate, polyoxyethylene (20) sorbitan monolaurate, polyoxyethylene (20) sorbitan monooleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, isopropyl myristate, glyceryl monooleate, glyceyl monostearate, glyceryl monoricinoleate, cetyl alcohol, stearyl alcohol, polyethylene glycol 400, cetyl pyridinium chloride, benzalkonium chloride, olive oil, glyceryl monolaurate, corn oil, cotton seed oil and sunflower seed oil. Preferred surfactants are lecithin, oleic acid and sorbitan trioleate. The amount of surfactant employed is desirably in the range of 0.0001 % to 50% w/w ratio relative to the medicament, in particular 0.05 to 5% w/w ratio.

Optionally, the formulations according to the invention may further comprise one or more stabilizers. The stabilizer is selected from the group consisting of glycin, glycine, alanine, valine, leucine, isoleucine, methionine, threonine, isovaline, phenylalanine, tyrosine, serine, histidine, tryptophan, proline, hydroxyproline, arginine, ornithine, asparagine, citrulline, aspartic acid, cysteine, glutamic acid, glutamine, lysine, hydroxylysine, N-acetyl-L-cysteine, phenylalanine, trans-4-hydroxy-L-proline, tyrosine, L-aspartyl-L-phenylalanine methylester and a mixture of any of the foregoing.

Optionally, the formulations according to the invention may further comprise one or more antioxidants. The antioxidant may be selected from the group consisting of tocopherol, deteroxime mesylate, methyl paraben, ethyl paraben and ascorbic acid and mixtures thereof. A preferred antioxidant is tocopherol.

The term "adduct" as used herein is meant to encompass a compound that is formed by the reaction of the medicament with the undesired leakage of a gaseous substance from a component of the medical device. Examples of adducts include a medicament-polymer adduct and Compound A. There are at least two possible mechanisms by which the medicament-formaldehyde adduct Compound A is formed. The first possibility is that the medicament-formaldehyde reaction is caused by direct contact between the medicament (TAA) and the plastic components of the DPI device that contain residual formaldehyde. The second possibility is that Compound A is formed from reaction between the medicament and gaseous formaldehyde in the inner local environment of the package, which has been released from the polyacetal components and accumulated in the local environment to a significant level due to the substantial impermeability of the package.

The term "gaseous substance" as used herein is meant to encompass any gaseous substance that is gradually emitted from the device and is capable of reacting with the medicament in the device to form a product e.g. an adduct. An example of such a gaseous substance is formaldehyde gas.

The term "adsorbent" as used herein is meant to encompass a substance which has the ability to condense or hold molecules of other substances on its surface or in its inner structure, an activity often referred as "adsorbing" or "absorbing". Examples of such adsorbents include activated carbon, alumina, bauxite, charcoal, zeolites, silica gel, molecular sieves, activated clays, bauxite, and mixtures thereof.

The present invention is not limited to any specific adsorbents. Although there are many different adsorbents and there are various trace gaseous substances, it is believed that any trace gaseous substance can be in principle entrapped by a properly-chosen adsorbent. Choosing a proper adsorbent for a given gaseous substance is well within the ordinary skill of the artisans in the field. They can make an initial choice based on their knowledge and experience (for example, weighing the factors such as the molecular size of the gaseous substance and the pore size of an adsorbent as well as electronic charges it carries) and then conduct tests to determine the actual effectiveness, and the effective amount, of the chosen adsorbent against a given gaseous substance. They may need to repeat the process until a proper adsorbent is found. One of the tests for finding an effective adsorbent against adduct formation is described herein and can be adopted by people skilled in the art to determine the actual effectiveness of any adsorbent, currently existing or to be developed in the future, against formation of medicament-adducts caused by any gaseous substances.

For preventing adduct formation caused by gaseous formaldehyde in medical devices comprising a medicament, Applicants have found that the most effective adsorbent material is molecular sieve with a pore size of about 10 Angstroms. Inclusion of 1 to 10 grams of the molecular sieve for example that supplied by AtoFina (Solihull, England) under the trade name Siliporite is found sufficient per package to prevent formation of medicament-polymer adducts in medical devices containing 5.8 mg/g TAA/lactose blend. More detailed technical information about molecular sieves and their other industrial uses can be found in the Hajdu article: Molecular Seives: Unique Moisture and Odor-Taste Control Material, D. Hajdu, T.J. Dangieri and S.R. Dunne, TAPPI Polym., Laminations Coat. Conf. (1999), Vol. 2, p. 655-662.

There are numerous ways in which the absorbent material can be present in the pharmaceutical product. For example, the adsorbent can be incorporated into a polymer mixture and manufactured into a plastic component of the medical device. Also, the adsorbent can be incorporated into a polymer mixture and manufactured into plastic sheeting used in the packaging of the device. The adsorbent can be incorporated into a polymer mixture in the same, or similar, manner as desiccant polymer mixtures disclosed in US Patent Nos. 5911937, 3245946, 4013566, 4407897, 4425410, 4464443 5078909 and 4792484. Although these patents disclose desiccants, it is foreseeable that the methods of manufacturing these plastics could be used to use to manufacture the adsorbent material used in the present invention. The adsorbent can also be in the form of an adsorbent incorporated in an adhesive (e.g. a self-adhesive patch or tape), in the same, or similar, manner as adhesive desiccants disclosed in US Patent No. 6103141.

The adsorbent material of the invention can also be in the form of an adsorbent in a porous sachet. Although it is not necessary to have a sachet to contain the adsorbent within the package, it is usually preferred. The adsorbent sachets are commercially available from many suppliers including Sud-Chemie (Middlewich, England). The sachet, with a "tea-bag" like appearance, is generally manufactured from synthetic fibers, such as polyamide or polyester fibers or blends thereof. Commercially available materials suitable for making adsorbent sachets include, for example, GDT-II from San-ei Corporation (Osaka, Japan) and Tyvek from Perfecseal (Londonderry N.Ireland U.K.). However, a suitable sachet may be in other convenient shapes or appearances and made from other permeable materials. Examples of adsorbents are selected from the group consisting of molecular sieves, activated clays, activated alumina, silica, zeolites, bauxites, and mixtures thereof. Preferably, 10 Å (Angstrom) molecular sieves. Molecular sieve material is commercially available from several manufacturers. For example AtoFina (Solihull, England) market a molecular sieve under the trade name of Siliporite. More detailed technical information about molecular sieves and their other industrial uses can be found in the Molecular Seives: Unique Moisture and Odor-Taste Control Material", D. Hajdu, T.J. Dangieri and S.R. Dunne, TAPPI Polym., Laminations Coat. Conf. (1999), Vol. 2, p. 655-662, which is incorporated herein by reference in its entirety.

The term "effective amount of an adsorbent" as used herein is intended to encompass the amount of an adsorbent material that is necessary to be effective in reducing formation of medicament adducts. The effective amount of adsorbent will depend on a number of factors, including the type of adsorbent and gas, the moisture content of the pharmaceutical product, and the amount of gaseous substance released. A person skilled in the art would readily be able to determine the effective amount of the adsorbent.

Due to the variety of forms in which the adsorbent can be present in the invention, the adsorbent can also be situated in a variety of places within the pharmaceutical product. For example, the adsorbent can be within a cavity in the medical device (i.e. housed in the device) e.g. the adsorbent can be situated inside the cap or inside the body of a dry-powder inhaler (see figure 3). Also, the adsorbent can be a component of the device e.g. the cap of a dry-powder inhaler can comprise an adsorbent polymer mixture (see figure 3). Also, the adsorbent can be affixed to the device in the form of an adhesive sticker/tape comprising the adsorbent. Furthermore, the adsorbent can be separate from the device in an enclosed volume within which the device is situated (see figure 2).

While there have been described and pointed out fundamental novel features of the invention as applied to a preferred embodiment thereof, it will be understood that various omissions and substitutions and changes, in the form and details of the packages, adsorbents, pharmaceutical products and methods illustrated, may be made by those skilled in the art without departing from the scope of the appended claims.

### Process of Finding an Effective Adsorbent against Adduct Formation

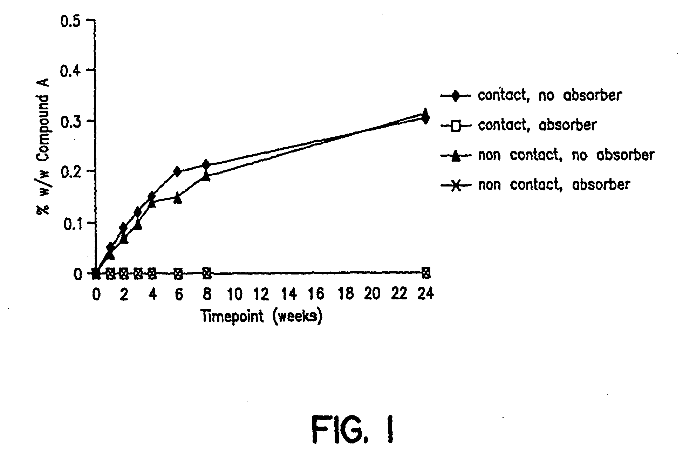

A study has been performed to determine an effective adsorbent against adduct formation, the result of which is summarized in figure 1. It showed that the adduct Compound A is formed not because of the direct contact between the medicament and the plastic components, but primarily because of the gaseous formaldehyde released from the plastic components and accumulated within the substantial impermeable local package environment. It further showed that the molecular sieve is an effective adsorbent in preventing the formation of Compound A.

The study was conducted in two groups: the contact group and non-contact group.

In the contact group, twenty-seven (27) samples were used, each comprising a dry-powder inhaler (DPI) sub assembly device core, assembled with only the upper mandrel and the powder chamber. The powder chambers were filled with 5.8 mg/g TAA/lactose blend. The samples were packaged with a laminated foil package, which provides a substantial impermeable enclosure. Thirteen (13) of the samples were packaged along with a molecular sieve as an adsorbent and the rest fourteen (14) samples did not include any adsorbent. The samples were stored at 40 °C/75%RH for 24 weeks. The blend from the power chamber was tested for the Compound A content and the adduct profile obtained initially and after storage for 1,2,3,4,6,8 and 24 weeks is shown in figure 1.

In the non-contact group, twenty-seven (27) samples were used. In each sample, a powder chamber filled with 5.8 mg/g TAA/lactose blend was contained in a breathable Tyvek bag and then placed in a sealed laminated foil package. Also placed in the sealed package was a polyacetal upper mandrel of the DPI sub assembly device core. Thus, the mandrel was in close proximity but not in direct contact with the blend itself. Of the samples, thirteen(13) included a molecular sieve within the sealed package and the remaining fourteen (14) did not. The samples were stored at 40 °C/75%RH for 24 weeks. The blend from the powder chamber was tested for the Compound A content and the adduct profile obtained initially and after storage for 1,2,3,4,6,8 and 24 weeks are shown in figure 1.

The above study result demonstrates that inclusion of an adsorbent inside the impermeable package is a simple and effective solution to the problem of medicament-polymer adduct formation occurred when medical devices comprising a medicament are packaged in impermeable packages. Particularly, molecular sieves are effective adsorbent materials against adduct formation caused by gaseous formaldehyde.

Although there are various types of adsorbent materials available and their effectiveness against any given gaseous substance varies considerably, it is understood that people of ordinary skill in the art can easily adopt the above-described study to determine the type and the amount of an adsorbent material that is effective in reducing formation of medicament adducts for any other types medical devices containing other different medicaments.

The invention is not limited by the embodiments described above which are presented as examples only but can be modified in various ways within the scope of protection defined by the appended claims.

## Claims

1. A pharmaceutical product comprising:
a) a medical device comprising a medicament and a component that gradually releases a gaseous substance, wherein said medicament is capable of interacting with the gaseous substance to form an adduct; and
b) an effective amount of an adsorbent or absorbent material capable of adsorbing or absorbing said gaseous substance, and reducing or preventing the formation of said adduct.

2. The pharmaceutical product according to claim 1, wherein the adsorbent or absorbent material is housed in the device.

3. The pharmaceutical product according to Claim 1, further comprising a sealed package having an enclosed volume within which the device and the adsorbent or absorbent material are situated;
wherein the sealed package is substantially impermeable to the gaseous substance; and wherein the gaseous substance is other than HFA (hydrofluoroalkane) propellant.

4. The pharmaceutical product according to claim 1, wherein the sealed package is substantially impermeable to moisture.

5. The pharmaceutical product according to any one of claims 1 to 4, wherein the device is selected from the group consisting of a syringe and a dry powder inhaler.

6. The pharmaceutical product according to any one of claims 1 to 5, wherein the device is a dry powder inhaler.

7. The pharmaceutical product according to any one of claims 1 to 6, wherein the medicament is an anti-inflammatory medicament used in the treatment of a respiratory disease.

8. The pharmaceutical product according to any one of claims 1 to 7, wherein the component undesirably releases the gaseous substance.

9. The pharmaceutical product according to any one of claims 1 to 8, wherein the component is a plastic element of a dry powder inhaler device.

10. The pharmaceutical product according to claim 9, wherein the plastic element comprises polyacetal material.

11. The pharmaceutical product according to any one of claims 1 to 10, wherein the gaseous substance is formaldehyde.

12. The pharmaceutical product according to any of claims 1 to 11, wherein the adsorbent or absorbent material is incorporated into a polymer mixture and manufactured into a plastic component of the medical device.

13. The pharmaceutical product according to any one of claims 1 to 11, wherein the adsorbent or absorbent material is incorporated into plastic sheeting used in the packaging of the device.

14. The pharmaceutical product according to any one of claims 1 to 11, wherein the adsorbent or absorbent material is incorporated in an adhesive.

15. The pharmaceutical product according to any one of claims 1 to 11, wherein the adsorbent or adsorbent material is in a porous sachet.

16. The pharmaceutical product according to any one of claims 1 to 15, wherein the adsorbent or absorbent material is selected from the group consisting of a molecular sieve, an activated clay, charcoal, activated alumina, silica, a zeolite, a bauxite, and a mixture thereof.

17. The pharmaceutical product according to any one of Claims 1 to 16, wherein the adsorbent or absorbent material is a 10 Å (Angstrom) molecular sieve.

18. The pharmaceutical product according to any one of claims 3 to 17, wherein the package is made of metal, glass, or plastic, and is selected from the group consisting of a bottle, a bag, a drum box, and an irregularly shaped container.

19. The pharmaceutical composition according to any one of claims 3 to 18, wherein the package is made of plastic.

20. The A pharmaceutical product according to any one of claims 3 to 19, wherein the package is a flexible laminate that comprises a polyester layer, an aluminum layer and a polyethylene layer, wherein the aluminum layer is between the polyester and polyethylene layers.

21. The pharmaceutical product according to any one of claims 3 to 20, wherein the package is hermetically sealed by heat-sealing, gluing, welding, brazing, mechanical closures or clamps, or compression.

22. The pharmaceutical product according to any one of claims 1 to 21, wherein the medicament is trimcinolone acetonide.

23. The pharmaceutical product according to any one of claims 1 to 22, wherein the adsorbent or absorbent material is in an amount sufficient to prevent the formation of an adduct.

24. The pharmaceutical product according to any one of claims 1 to 23, wherein the adduct is of the formula:

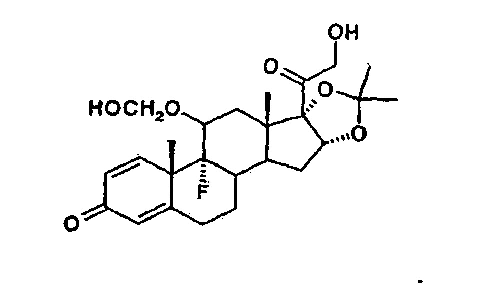

25. A method for preventing the formation of an adduct in a pharmaceutical product due to a chemical reaction between the medicament and a gaseous substances, wherein the pharmaceutical product comprises:
a) a medical device comprising a medicament capable of forming an adduct and a component that gradually releases a gaseous substance; and
b) an effective amount of an adsorbent or absorbent material capable of adsorbing or absorbing said gaseous substance, and reducing or preventing the formation of said adduct,
wherein the method comprises the steps of:
(i) positioning an effective amount of the adsorbent or absorbent material and the medical device within a sealable package;
(ii) sealing the package so that the medical device and adsorbent are in an enclosed volume within the package; and
(iii) adsorbing or absorbing any leakage of the gaseous substance from the component so as to reduce or prevent the formation of the adduct

26. The method according to claim 25, wherein the adsorbent or absorbent material is housed in the device.

27. The method according to any one of claims 25 to 26, wherein the sealed package is substantially impermeable to the gaseous substance; and wherein the gaseous substance is other than HFA (hydrofluoroalkane) propellant.

28. The method according to any one of claims 25 to 27, wherein the medicament is an anti-inflammatory medicament used in the treatment of a respiratory disease.

29. The method according to any one of claims 25 to 28, wherein the gaseous substance is formaldehyde.

30. The method according to any one of claims 25 to 29, wherein the adsorbent or absorbent material is incorporated into a polymer mixture and manufactured into a plastic component of the medical device.

31. The method according to any one of claims 25 to 29, wherein the adsorbent or absorbent material is incorporated into plastic sheeting used in the packaging of the device.

32. The method according to any one of claims 25 to 29, wherein the adsorbent or absorbent material is incorporated in an adhesive.

33. The method according to any one of claims 25 to 29, wherein the adsorbent or absorbent material is in a porous sachet.

34. The method according to any one of claims 25 to 33, wherein the adsorbent or absorbent material is selected from the group consisting of a molecular sieve, an activated day, charcoal, activated alumina, silica, a zeolite, a bauxite, and a mixture thereof.

35. The method according to any one of claims 25 to 34, wherein the adsorbent or absorbent material is a 10 Å (Angstrom) molecular sieve.

36. The method according to any one of claims 25 to 35, wherein the medicament is triamcinolone acetonide.

37. The method according to any one of claims 25 to 36, wherein the adsorbent or absorbent material is in an amount sufficient to prevent formation of an adduct.

38. The method according to any one of claims 25 to 37, wherein the adduct is of the formula:

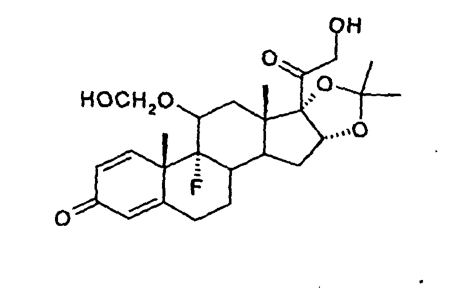

## Patentansprüche

1. Pharmazeutisches Produkt, umfassend:
a) eine medizinische Einheit, umfassend ein Medikament und eine Komponente, welche einen gasförmigen Stoff allmählich freisetzt, wobei das Medikament mit dem gasförmigen Stoff wechselwirken kann, um ein Addukt zu bilden; und
b) eine wirksame Menge eines Adsorbtionsmittels oder absorbierenden Materials, das den gasförmigen Stoff adsorbieren oder absorbieren und die Bildung des Addukts verringern oder verhindern kann.

2. Pharmazeutisches Produkt gemäß Anspruch 1, wobei das Adsorptionsmittel oder absorbierende Material in der Einheit angeordnet ist.

3. Pharmazeutisches Produkt gemäß Anspruch 1, ferner umfassend eine verschließbare Verpackung mit einem darin eingeschlossenen Volumen, in welchem die Einheit und das Adsorptionsmittel oder absorbierende Material angeordnet sind;
wobei die verschlossene Verpackung im Wesentlichen undurchlässig für den gasförmigen Stoff ist; und wobei der gasförmige Stoff von HFA-Treibgas (Hydrofluoralkan-Treibgas) verschieden ist.

4. Pharmazeutisches Produkt gemäß Anspruch 1, wobei die verschlossene Verpackung im Wesentlichen undurchlässig für Feuchtigkeit ist.

5. Pharmazeutisches Produkt gemäß einem der Ansprüche 1 bis 4, wobei die Einheit ausgewählt ist aus der Gruppe, bestehend aus einer Spritze und einem Trockenpulver-Inhalator.

6. Pharmazeutisches Produkt gemäß einem der Ansprüche 1 bis 5, wobei die Einheit ein Trockenpulver-Inhalator ist.

7. Pharmazeutisches Produkt gemäß einem der Ansprüche 1 bis 6, wobei das Medikament ein entzündungshemmendes Medikament ist, das bei der Behandlung einer Atemwegserkrankung verwendet wird.

8. Pharmazeutisches Produkt gemäß einem der Ansprüche 1 bis 7, wobei die Komponente unerwünschterweise den gasförmigen Stoff freisetzt.

9. Pharmazeutisches Produkt gemäß einem der Ansprüche 1 bis 8, wobei die Komponente ein Kunststoffelement einer Trockenpulver-Inhalatoreinheit ist.

10. Pharmazeutisches Produkt gemäß Anspruch 9, wobei das Kunststoffelement Polyacetalmaterial umfasst.

11. Pharmazeutisches Produkt gemäß einem der Ansprüche 1 bis 10, wobei der gasförmige Stoff Formaldehyd ist.

12. Pharmazeutisches Produkt gemäß einem der Ansprüche 1 bis 11, wobei das Adsorptionsmittel oder absorbierende Material einem Polymergemisch einverleibt und zu einer Kunststoffkomponente der medizinischen Einheit verarbeitet ist.

13. Pharmazeutisches Produkt gemäß einem der Ansprüche 1 bis 11, wobei das Adsorptionsmittel oder absorbierende Material einer Kunststofffolie einverleibt ist, die bei der Verpackung der Einheit verwendet wird.

14. Pharmazeutisches Produkt gemäß einem der Ansprüche 1 bis 11, wobei das Adsorptionsmittel oder absorbierende Material einem Klebstoff einverleibt ist.

15. Pharmazeutisches Produkt gemäß einem der Ansprüche 1 bis 11, wobei sich das Adsorptionsmittel oder absorbierende Material in einem porösen Kissen befindet.

16. Pharmazeutisches Produkt gemäß einem der Ansprüche 1 bis 15, wobei das Adsorptionsmittel oder absorbierende Material ausgewählt ist aus der Gruppe, bestehend aus einem Molekularsieb, einem aktivierten Ton, Aktivkohle, aktiviertem Aluminiumoxid, Silica, einem Zeolit, einem Bauxit und einem Gemisch davon.

17. Pharmazeutisches Produkt gemäß einem der Ansprüche 1 bis 16, wobei das Adsorptionsmittel oder absorbierende Material ein 10 Å (Angström)-Molekularsieb ist.

18. Pharmazeutisches Produkt gemäß einem der Ansprüche 3 bis 17, wobei die Verpackung aus Metall, Glas oder Kunststoff hergestellt und aus der Gruppe, bestehend aus einer Flasche, einer Tasche, einer Trommelschachtel und einem unregelmäßig geformten Behälter, ausgewählt ist.

19. Pharmazeutisches Produkt gemäß einem der Ansprüche 3 bis 18, wobei die Verpackung aus Kunststoff hergestellt ist.

20. Pharmazeutisches Produkt gemäß einem der Ansprüche 3 bis 19, wobei die Verpackung ein flexibles Laminat ist, welches eine Polyesterschicht, eine Aluminiumschicht und eine Polyethylenschicht umfasst, wobei die Aluminiumschicht zwischen der Polyester- und der Polyethylenschicht angeordnet ist.

21. Pharmazeutisches Produkt gemäß einem der Ansprüche 3 bis 20, wobei die Verpackung durch Wärmeversiegeln, Kleben, Schweißen, Löten, mechanische Verschlüsse oder Klammern, oder Druck luftdicht verschlossen ist.

22. Pharmazeutisches Produkt gemäß einem der Ansprüche 1 bis 21, wobei das Medikament Triamcinolonacetonid ist.

23. Pharmazeutisches Produkt gemäß einem der Ansprüche 1 bis 22, wobei das Adsorptionsmittel oder absorbierende Material in einer Menge vorliegt, die zum Verhindern der Bildung eines Addukts ausreicht.

24. Pharmazeutisches Produkt gemäß einem der Ansprüche 1 bis 23, wobei das Addukt folgende Formel aufweist:

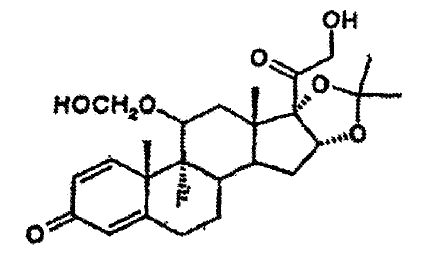

25. Verfahren zum Verhindern der Bildung eines Addukts in einem pharmazeutischen Produkt durch eine chemische Umsetzung zwischen dem Medikament und einem gasförmigen Stoff, wobei das pharmazeutische Produkt folgendes umfasst:
a) eine medizinische Einheit, umfassend ein Medikament, das ein Addukt bilden kann, und eine Komponente, welche einen gasförmigen Stoff allmählich freisetzt; und
b) eine wirksame Menge eines Adsorbtionsmittels oder absorbierenden Materials, das den gasförmigen Stoff adsorbieren oder absorbieren und die Bildung des Addukts verringern oder verhindern kann,
wobei das Verfahren folgende Schritte umfasst:
(i) Anordnen einer wirksamen Menge des Adsorbtionsmittels oder absorbierenden Materials und der medizinischen Einheit in einer verschließbaren Verpackung;
(ii) Verschließen der Verpackung, so dass sich die medizinische Einheit und das Adsorptionsmittel in einem eingeschlossenen Volumen innerhalb der Verpackung befinden; und
(iii) Adsorbieren oder Absorbieren aller Leckage des gasförmigen Stoffs aus der Komponente, um die Bildung des Addukts zu verringern oder zu verhindern.

26. Verfahren gemäß Anspruch 25, wobei das Adsorbtionsmittel oder absorbierende Material in der Einheit angeordnet ist.

27. Verfahren gemäß einem der Ansprüche 25 bis 26, wobei die verschlossene Verpackung im Wesentlichen undurchlässig für den gasförmigen Stoff ist; und wobei der gasförmige Stoff von HFA-Treibgas (Hydrofluoralkan-Treibgas) verschieden ist.

28. Verfahren gemäß einem der Ansprüche 25 bis 27, wobei das Medikament ein entzündungshemmendes Medikament ist, das bei der Behandlung einer Atemwegserkrankung verwendet wird.

29. Verfahren gemäß einem der Ansprüche 25 bis 28, wobei der gasförmige Stoff Formaldehyd ist.

30. Verfahren gemäß einem der Ansprüche 25 bis 29, wobei das Adsorptionsmittel oder absorbierende Material einem Polymergemisch einverleibt und zu einer Kunststoffkomponente der medizinischen Einheit verarbeitet ist.

31. Verfahren gemäß einem der Ansprüche 25 bis 29, wobei das Adsorptionsmittel oder absorbierende Material einer Kunststofffolie einverleibt ist, die bei der Verpackung der Einheit verwendet wird.

32. Verfahren gemäß einem der Ansprüche 25 bis 29, wobei das Adsorptionsmittel oder absorbierende Material einem Klebstoff einverleibt ist.

33. Verfahren gemäß einem der Ansprüche 25 bis 29, wobei sich das Adsorptionsmittel oder absorbierende Material in einem porösen Kissen befindet.

34. Verfahren gemäß einem der Ansprüche 25 bis 33, wobei das Adsorptionsmittel oder absorbierende Material ausgewählt ist aus der Gruppe, bestehend aus einem Molekularsieb, einem aktivierten Ton, Aktivkohle, aktiviertem Aluminiumoxid, Silica, einem Zeolit, einem Bauxit und einem Gemisch davon.

35. Verfahren gemäß einem der Ansprüche 25 bis 34, wobei das Adsorptionsmittel oder absorbierende Material ein 10 Å (Angström)-Molekularsieb ist.

36. Verfahren gemäß einem der Ansprüche 25 bis 35, wobei das Medikament Triamcinolonacetonid ist.

37. Verfahren gemäß einem der Ansprüche 25 bis 36, wobei das Adsorptionsmittel oder absorbierende Material in einer Menge vorliegt, die zum Verhindern der Bildung eines Addukts ausreicht.

38. Verfahren gemäß einem der Ansprüche 25 bis 37, wobei das Addukt folgende Formel aufweist:

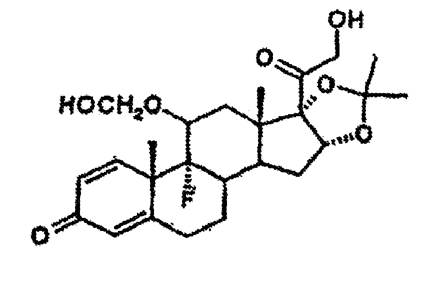

## Revendications

1. Produit pharmaceutique comprenant:
a) un dispositif médical comprenant un médicament et un composant qui libère progressivement une substance gazeuse, dans lequel ledit médicament est capable d'interagir avec la substance gazeuse pour former un composé d'addition; et
b) une quantité efficace d'un matériau adsorbant ou absorbant capable d'adsorber ou d'adsorber ladite substance gazeuse, et de réduire ou d'empêcher la formation dudit composé d'addition.

2. Produit pharmaceutique selon la revendication 1, dans lequel le matériau adsorbant ou absorbant est logé dans le dispositif.

3. Produit pharmaceutique selon la revendication 1, comprenant en outre un emballage étanche présentant un volume fermé à l'intérieur duquel sont situés le dispositif et le matériau adsorbant ou absorbant;
dans lequel l'emballage étanche est essentiellement imperméable à la substance gazeuse; et dans lequel la substance gazeuse est autre qu'un propulseur HFA (hydrofluoroalcane).

4. Produit pharmaceutique selon la revendication 1, dans lequel l'emballage étanche est essentiellement imperméable à l'humidité.

5. Produit pharmaceutique selon l'une quelconque des revendications 1 à 4, dans lequel le dispositif est choisi dans le groupe constitué par une seringue et un inhalateur de poudre sèche.

6. Produit pharmaceutique selon l'une quelconque des revendications 1 à 5, dans lequel le dispositif est un inhalateur de poudre sèche.

7. Produit pharmaceutique selon l'une quelconque des revendications 1 à 6, dans lequel le médicament est un médicament anti-inflammatoire employé dans le traitement d'une maladie respiratoire.

8. Produit pharmaceutique selon l'une quelconque des revendications 1 à 7, dans lequel le composant libère, de façon indésirable, la substance gazeuse.

9. Produit pharmaceutique selon l'une quelconque des revendications 1 à 8, dans lequel le composant est un élément en matière plastique d'un dispositif inhalateur de poudre sèche.

10. Produit pharmaceutique selon la revendication 9, dans lequel l'élément en matière plastique comprend un matériau en polyacétal.

11. Produit pharmaceutique selon l'une quelconque des revendications 1 à 10, dans lequel la substance gazeuse est du formaldéhyde.

12. Produit pharmaceutique selon l'une quelconque des revendications 1 à 11, dans lequel le matériau adsorbant ou absorbant est incorporé dans un mélange de polymères et manufacturé pour former un composant en matière plastique du dispositif médical.

13. Produit pharmaceutique selon l'une quelconque des revendications 1 à 11, dans lequel le matériau adsorbant ou absorbant est incorporé dans une feuille de matière plastique utilisée pour emballer le dispositif.

14. Produit pharmaceutique selon l'une quelconque des revendications 1 à 11, dans lequel le matériau adsorbant ou absorbant est incorporé dans un adhésif.

15. Produit pharmaceutique selon l'une quelconque des revendications 1 à 11, dans lequel le matériau adsorbant ou absorbant est dans un sachet poreux.

16. Produit pharmaceutique selon l'une quelconque des revendications 1 à 15, dans lequel le matériau adsorbant ou absorbant est choisi dans le groupe constitué par un tamis moléculaire, une argile activée, du charbon, de l'alumine activée, de la silice, une zéolite, une bauxite, et un mélange de ceux-ci.

17. Produit pharmaceutique selon l'une quelconque des revendications 1 à 16, dans lequel le matériau adsorbant ou absorbant est un tamis moléculaire de 10 Å (Angström) .

18. Produit pharmaceutique selon l'une quelconque des revendications 3 à 17, dans lequel l'emballage est fait de métal, de verre ou de matière plastique, et est choisi dans le groupe constitué par une bouteille, un sac, un bidon, et un récipient de forme irrégulière.

19. Composition pharmaceutique selon l'une quelconque des revendications 3 à 18, dans laquelle l'emballage est fait de matière plastique.

20. Produit pharmaceutique selon l'une quelconque des revendications 3 à 19, dans lequel l'emballage est un stratifié flexible qui comprend une couche de polyester, une couche d'aluminium et une couche de polyéthylène, dans lequel la couche d'aluminium est placée entre la couche de polyester et la couche de polyéthylène.

21. Produit pharmaceutique selon l'une quelconque des revendications 3 à 20, dans lequel l'emballage est rendu étanche par thermosoudage, collage, soudage, brasage, au moyen de fermetures mécaniques ou de pinces, ou par compression.

22. Produit pharmaceutique selon l'une quelconque des revendications 1 à 21, dans lequel le médicament est le triamcinolone acétonide.

23. Produit pharmaceutique selon l'une quelconque des revendications 1 à 22, dans lequel le matériau adsorbant ou absorbant est en quantité suffisante pour empêcher la formation d'un composé d'addition.

24. Produit pharmaceutique selon l'une quelconque des revendications 1 à 23, dans lequel le composé d'addition a pour formule:

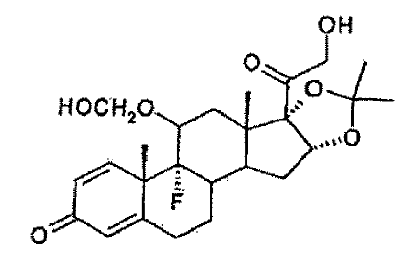

25. Procédé pour empêcher la formation d'un composé d'addition dans un produit pharmaceutique due à une réaction chimique entre le médicament et une substance gazeuse, dans lequel le produit pharmaceutique comprend:
a) un dispositif médical comprenant un médicament capable de former un composé d'addition et un composant qui libère progressivement une substance gazeuse; et
b) une quantité efficace d'un matériau adsorbant ou absorbant capable d'adsorber ou d'absorber ladite substance gazeuse, et de réduire ou d'empêcher la formation dudit composé d'addition,
dans lequel le procédé comprend les étapes consistant à:
(i) placer une quantité efficace du matériau adsorbant ou absorbant et le dispositif médical à l'intérieur d'un emballage pouvant être fermé de façon étanche;
(ii) fermer de façon étanche l'emballage de telle sorte que le dispositif médical et l'adsorbant soient dans un volume fermé à l'intérieur de l'emballage; et
(iii) adsorber ou absorber les fuites éventuelles de la substance gazeuse provenant du composant de manière à réduire ou à empêcher la formation du composé d'addition.

26. Procédé selon la revendication 25, dans lequel le matériau adsorbant ou absorbant est logé dans le dispositif.

27. Procédé selon l'une quelconque des revendications 25 à 26, dans lequel l'emballage étanche est essentiellement imperméable à la substance gazeuse; et dans lequel la substance gazeuse est autre qu'un propulseur HFÅ (hydrofluoroalcane).

28. Procédé selon l'une quelconque des revendications 25 à 27, dans lequel le médicament est un médicament anti-inflammatoire employé dans le traitement d'une maladie respiratoire.

29. Procédé selon l'une quelconque des revendications 25 à 28, dans lequel la substance gazeuse est du formaldéhyde.

30. Procédé selon l'une quelconque des revendications 25 à 29, dans lequel le matériau adsorbant ou absorbant est incorporé dans un mélange de polymères et manufacturé pour former un composant en matière plastique du dispositif médical.

31. Procédé selon l'une quelconque des revendications 25 à 29, dans lequel le matériau adsorbant ou absorbant est incorporé dans une feuille de matière plastique utilisée pour emballer le dispositif.

32. Procédé selon l'une quelconque des revendications 25 à 29, dans lequel le matériau adsorbant ou absorbant est incorporé dans un adhésif.

33. Procédé selon l'une quelconque des revendications 25 à 29, dans lequel le matériau adsorbant ou absorbant est dans un sachet poreux.

34. Procédé selon l'une quelconque des revendications 25 à 33, dans lequel le matériau adsorbant ou absorbant est choisi dans le groupe constitué par un tamis moléculaire, une argile activée, du charbon, de l'alumine activée, de la silice, une zéolite, une bauxite, et un mélange de ceux-ci.

35. Procédé selon l'une quelconque des revendications 25 à 34, dans lequel le matériau adsorbant ou absorbant est un tamis moléculaire de 10 Å (Angström) .

36. Procédé selon l'une quelconque des revendications 25 à 35, dans lequel le médicament est le triamcinolone acétonide.

37. Procédé selon l'une quelconque des revendications 25 à 36, dans lequel le matériau adsorbant ou absorbant est en quantité suffisante pour empêcher la formation d'un composé d'addition.

38. Procédé selon l'une quelconque des revendications 25 à 37, dans lequel le composé d'addition a pour formule:
